# EUROPÄISCHE PATENTSCHRIFT

(11) **EP 0 793 651 B1**
(45) Veröffentlichungstag und Bekanntmachung des Hinweises auf die Patenterteilung: **19.09.2001**
(21) Anmeldenummer: 95940996.2
(22) Anmeldetag: 22.11.1995
(51) Int. Cl.: C07D 201/12

(54) **VERFAHREN ZUR HERSTELLUNG VON CAPROLACTAM**
PROCESS FOR PREPARING CAPROLACTAM
PROCEDE DE PREPARATION DE CAPROLACTAME

(30) Priorität: 01.12.1994 DE 4442727
(43) Veröffentlichungstag der Anmeldung: 10.09.1997
(73) Patentinhaber: BASF AKTIENGESELLSCHAFT, 67056 Ludwigshafen (DE)
(72) Erfinder: FUCHS, Eberhard, D-67227 Frankenthal (DE); WITZEL, Tom, D-67069 Ludwigshafen (DE)
(86) Internationale Anmeldenummer: EP9504596
(87) Internationale Veröffentlichungsnummer: WO9616937

(56) Entgegenhaltungen:
- EP-A- 0 209 021
- EP-A- 0 529 470

## Beschreibung

Die vorliegende Erfindung betrifft ein verbessertes Verfahren zur Herstellung von Caprolactam durch Spaltung von Oligomeren und/ oder Polymeren, enthaltend im wesentlichen die wiederkehrende Einheit -[-N(H)-(CH₂)₅-C(O)-]-, in Gegenwart eines Katalysators bei erhöhter Temperatur.

Die Spaltung von Polyamid-6 (Polycaprolactam) zu Caprolactam führt man meistens unter der Einwirkung saurer oder basischer Katalysatoren bei erhöhter Temperatur häufig unter Einwirkung von Wasserdampf im Niederdruckbereich durch. In der Chem. Ing. Techn. 45 (1973) 1510 wird die technische Durchführung eines Spaltverfahrens mit überhitztem Wasserdampf beschrieben, wobei jedoch zur Aufarbeitung die Aufkonzentrierung einer Caprolactam/Wasser-Lösung erforderlich ist.

In der EP-A 209,021 wird die Spaltung in einem Aluminiumoxid-Wirbelbett beschrieben. Nebenproduktbildungen und Deaktivierungen durch Verkleben der Katalysatorschüttung sind häufig die Folge. Im Verfahren nach der EP 529 470 wird als Katalysator zur Polyamid-6-Spaltung Kaliumcarbonat zugesetzt, wobei man die Reaktion bei 250 bis 320°C unter gleichzeitigem Abdestillieren des Caprolactams im Vakuum durchführt.

Alle bisherigen Verfahren zur Spaltung von Polyamid-6 zu Caprolactam weisen als Nachteile die energetisch sehr aufwendige Abtrennung großer Wassermengen sowie das Entfernen von Katalysatoren wie Phosphorsäuren und deren Salze, Kaliumcarbonat oder Alkalimetalloxide auf.

Aufgabe der vorliegenden Erfindung war es daher, ein verbessertes Verfahren zur Herstellung von Caprolactam, ausgehend von Oligomeren und/oder Polymeren von Caprolactam, zur Verfügung zu stellen, das die genannten Nachteile nicht aufweist.

Demgemäß wurde ein Verfahren zur Herstellung von Caprolactam durch Spaltung von Oligomeren oder Polymeren, enthaltend im wesentlichen die wiederkehrende Einheit -[-N(H)-(CH₂)₅-C(O)-]-,in Gegenwart eines Katalysators bei erhöhter Temperatur gefunden, indem man die Spaltung in flüssiger Phase in Gegenwart eines heterogenen Katalysators und eines organischen Lösungsmittels durchführt.

Erfindungsgemäß geht man von Oligomeren und/oder Polymeren aus, die die wiederkehrende Einheit -[-N(H)-(CH₂)₅-C(O)-]- enthalten. Bevorzugt setzt man Polycaprolactam sowie Oligomere von Caprolactam ein sowie Copolymere von Caprolactam beispielsweise erhalten durch Polymerisation von Caprolactam in Gegenwart von Hexamethylendiamin und Terephthalsäure (beispielsweise beschrieben in der EP-A 299 444).

Des weiteren kann man Oligo- und/oder Polymere des Caprolactams einsetzen, die entsorgt werden sollen, z.B. aus Abfällen, die bei der Herstellung von Caprolactam oder Polycaprolactam oder dessen Verarbeitung zu Fäden, Folien, Spritzguß- oder Extrusionsteilen anfallen, ferner geformte Gebrauchsgegenstände wie Folien, Verpackungen, Gewebe, Fäden, Fasern, extrudierte Teile. Zweckmäßig zerkleinert man dabei, beispielsweise durch Mahlen, die zu spaltenden Gegenstände vor der Spaltung.

Die Umsetzung nimmt man erfindungsgemäß in flüssiger Phase bei Temperaturen im Bereich von 140 bis 320, vorzugsweise von 160 bis 300°C vor. Den Druck wählt man im Bereich von 0,5 bis 25, vorzugsweise von 5 bis 20 MPa, wobei darauf zu achten ist, daß das Reaktionsgemisch unter den angewandten Bedingungen flüssig ist. Die Verweilzeiten wählt man im allgemeinen im Bereich von 5 bis 300, vorzugsweise von 7 bis 180 min.

Die Spaltung nimmt man erfindungsgemäß ohne Zusatz von Wasser vor. In einer bevorzugten Ausführungsform setzt man Wasser ein und zwar pro mol der wiederkehrenden Einheit -[-N(H)-(CH₂)₅-C(O)-]- werden bevorzugt von 0,5 bis 20, insbesondere von 1 bis 5 mol Wasser eingesetzt.

Erfindungsgemäß wird die Spaltung in Gegenwart eines organischen Lösungsmittels durchgeführt, wobei man vorteilhaft die Oligomeren bzw. die Polymeren in Form einer 1 bis 50, insbesondere einer 5 bis 40, besonders vorzugsweise einer 5 bis 25 gew.-%igen Lösung in dem organischen Lösungsmittel einsetzt, wobei man besonders bevorzugt eine Mischung aus Wasser und dem organischen Lösungsmittel mit den zuvor genannten Gewichtsprozentangaben für die Oligo- und/oder Polymeren einsetzt.

Als organische Lösungsmittel seien beispielhaft genannt:
C₁-C₁₀-Alkanole wie Methanol, Ethanol, n-, i-Propanol, n-, i-, sek.-Butanol, n-Pentanol, n-Hexanol, n-Heptanol, n-Octanol, n-Nonanol und n-Decanol, insbesondere die C₁-C₄-Alkanole wie Methanol, Ethanol, n-, i-Propanol, n-, i-, sek.-Butanol, besonders bevorzugt Methanol, Ethanol, n-Propanol und n-Butanol.
Polyole wie Diethylenglykol und Tetraethylenglykol, bevorzugt Tetraethylenglylkol;
Kohlenwasserstoffe wie Petrolether, Benzol, Toluol, Xylole;
Lactame wie Pyrrolidon und Caprolactam sowie substituierte N-C₁-C₄-Alkyllactame wie N-Methylcaprolactam, N-Ethylcaprolactam und N-Methyl-pyrrolidon.

In einer weiteren Ausführungsform kann man dem Reaktionsgemisch von 0 bis 5, bevorzugt von 0,1 bis 2 Gew.-% Ammoniak und/oder Wasserstoff und/oder Stickstoff zusetzen.

Als heterogene Katalysatoren können beispielsweise verwendet werden: saure, basische oder amphotere Oxide der Elemente der zweiten, dritten oder vierten Hauptgruppe wie Calciumoxid, Magnesiumoxid, Boroxid, Aluminiumoxid, Zinnoxid oder Siliciumoxid als pyrogen hergestelltes Siliciumdioxid, als Kieselgel, Kieselgur, Quarz oder Mischungen davon, weiterhin Oxide von Metallen der zweiten bis sechsten Nebengruppe des Periodensystems wie Titanoxid, amorph, als Anatas oder Rutil, Zirkonoxid, Zinkoxid, Manganoxid oder Mischungen davon. Ebenfalls verwendbar sind beispielsweise Oxide der Lanthaniden und Actiniden wie Ceroxid, Thoriumoxid, Praseodymoxid, Samariumoxid, Neodymoxid, Seltenerd-Mischoxide oder Mischungen davon mit den zuvor genannten Oxiden. Weitere Katalysatoren können beispielsweise sein: Vanadiumoxid, Nioboxid, Eisenoxid, Chromoxid, Molybdänoxid, Wolframoxid oder Mischungen davon. Mischungen dieser Oxide mit denen der zweiten, dritten und vierten Hauptgruppe sind ebenfalls möglich. Auch einige Sulfide, Selenide und Telluride wie Zink-Tellurid, Zink-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weitere Katalysatoren sind beispielsweise Zeolithe, Phosphate und Heteropolysäuren sowie saure und alkalische Ionenaustauscher wie Naphion®.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Die Katalysatoren können je nach der Zusammensetzung des Katalysators als Vollkontakt oder Trägerkatalysator verwendet werden. So kann beispielsweise Titandioxid als Titandioxid-Strang oder als auf einen Träger in dünner Schicht aufgebrachtes Titandioxid eingesetzt werden. Zum Aufbringen von Titandioxid auf einen Träger wie Siliciumdioxid, Aluminiumoxid oder Zirkondioxid sind nach bisherigen Beobachtungen alle in der Literatur beschriebenen Methoden verwendbar. So kann eine dünne Titandioxid-Schicht durch Hydrolyse von Titanorganylen wie Titan-isopropylat oder Titanbutylat, oder durch Hydrolyse von Titantetrachlorid oder anderen anorganischen Titan-haltigen Verbindungen aufgebracht werden. Auch Titanoxid-haltige Sole sind einsetzbar.

Der Vorteil des erfindungsgemäßen Verfahrens liegt darin, daß im Gegensatz zu Verfahren aus dem Stand der Technik energetisch sehr aufwendige Abtrennung großer Wassermengen sowie das Entfernen von Katalysatoren wie Phosphorsäuren und deren Salze, Kaliumcarbonat oder Alkalimetalloxide entfallen.

### Beispiele

### Beispiel 1

In einen geheizten Rohrreaktor von 25 ml Inhalt (Durchmesser 6 mm; Länge 800 mm), der mit Titandioxid (Anatas) in Form von 1,5 mm Strängen gefüllt war, wurde bei 100 bar eine 10 gew.-%ige Lösung von 6-Aminocapronitril (ACN) in 6,4 Gew.-% Wasser und 83,6 Gew.-% Ethanol bei einer Temperatur von 200°C geleitet. Die Verweilzeit betrug 30 min. Der den Reaktor verlassende Produktstrom wurde gaschromatographisch und hochdruckflüssigchromatographisch analysiert. Umsatz an Caprolactam: 100 %, Selektivität an Caprolactam: 88 %.

Die so erhaltene Mischung wurde einer Destillation (Temperatur: 110°C, Druck: 0,1 mbar) zur Gewinnung von Caprolactam unterworfen, wobei als Sumpf eine Mischung bestehend aus 9 Gew.-% Oligo- und Polymeren, 1 Gew.-% Caprolactam, 6,4 Gew.-% Wasser sowie 83,4 Gew.-% Ethanol verblieben.

### Beispiel 2

(a) In einen auf 230°C geheizten Rohrreaktor von 25 ml Inhalt (Durchmesser 6 mm, Länge 800 mm), der mit Titanoxid (Anatas) in Form von 1,5 mm Strängen gefüllt war, wurden bei 100 bar 60 ml/h des Sumpfes aus Beispiel 1 zugeführt. Die Verweilzeit im Reaktor betrug 15 min.

Der Umsatz zu Caprolactam, bezogen auf die eingesetzte Menge an Oligo- und Polymere mit der wiederkehrenden Einheit -[-N(H)-(CH₂)₅-C(O)-]- betrug 53%.

### Beispiele 3, 4 und 5

Beispiel 2 wurde wiederholt mit den in untenstehender Tabelle aufgeführten Unterschieden.

**Tabelle**

| Bsp. | Temp. [°C] | Verweilzeit [min] | Umsatz [%] |
|---|---|---|---|
| 3 | 220 | 15 | 50 |
| 4 | 220 | 30 | 62 |
| 5 | 250 | 10 | 63 |

## Patentansprüche

1. Verfahren zur Herstellung von Caprolactam durch Spaltung von Oligomeren und/oder Polymeren, enthaltend im wesentlichen die wiederkehrende Einheit -[-N(H) - (CH₂)₅-C(O)-]-, in Gegenwart eines Katalysators bei erhöhter Temperatur, **dadurch gekennzeichnet, daß** man die Spaltung in Gegenwart eines heterogenen Katalysators und eines organischen Lösungsmittels bei einer Temperatur im Bereich von 140 bis 320°C und einem Druck im Bereich von 0,5 bis 25 MPa durchführt, wobei das Reaktionsgemisch unter den angewandten Bedingungen flüssig ist.

2. Verfahren nach Anspruch 1, **dadurch gekennzeichnet, daß** man die Reaktion bei einer Konzentration der Oligo- und/oder Polymeren im Bereich von 5 bis 50 Gew.-% durchführt.

3. Verfahren nach den Ansprüchen 1 oder 2, **dadurch gekennzeichnet, daß** man die Verweilzeit im Bereich von 5 bis 300 min wählt.

4. Verfahren nach den Ansprüchen 1 bis 3, **dadurch gekennzeichnet, daß** man als organisches Lösungsmittel ein C₁-C₄-Alkanol oder Tetraethylenglykol einsetzt.

5. Verfahren gemäß den Ansprüchen 1 bis 4, **dadurch gekennzeichnet, daß** man die Spaltung in Gegenwart von Wasser durchführt.

## Claims

1. A process for the preparation of caprolactam by cleaving oligomers or polymers containing essentially the repeating unit -[-N(H)-(CH₂)₅-C(O)-]- in the presence of a catalyst at elevated temperatures, wherein the cleavage is carried out in the presence of a heterogeneous catalyst and of an organic solvent at from 140 to 320°C and from 0.5 to 25 MPa, the reaction mixture being liquid under the conditions used.

2. A process as claimed in claim 1, wherein the reaction is carried out at a concentration of the oligomers or polymers of from 5 to 50% by weight.

3. A process as claimed in claim 1 or 2, wherein the residence time is chosen to be from 5 to 300 minutes.

4. A process as claimed in any of claims 1 to 3, wherein the organic solvent used is a C₁-C₄-alkanol or tetraethylene glycol.

5. A process as claimed in any of claims 1 to 4, wherein the cleavage is carried out in the presence of water.

## Revendications

1. Procédé de préparation de caprolactame par craquage d'oligomères et/ou de polymères, contenant essentiellement l'unité répétitive -[-N(H)-(CH₂)₅-C(O)-]-, en présence d'un catalyseur et à température élevée, **caractérisé en ce que** l'on entreprend le craquage en présence d'un catalyseur hétérogène et d'un solvant organique, à une température de l'ordre de 140 à 320°C et une pression de l'ordre de 0,5 à 25 MPa, le mélange réactionnel étant liquide aux conditions appliquées.

2. Procédé selon la revendication 1, **caractérisé en ce que** la réaction est entreprise avec une concentration des oligomères et/ou polymères de l'ordre de 5 à 50% en poids.

3. Procédé selon la revendication 1 ou 2, **caractérisé en ce que** l'on choisit un temps de séjour de l'ordre de 5 à 300 minutes.

4. Procédé selon les revendications 1 à 3, **caractérisé en ce que** l'on met en oeuvre, en tant que solvant organique, un alcanol en C₁-C₄ ou du tétraéthylène glycol.

5. Procédé selon les revendications 1 à 4, **caractérisé en ce que** l'on entreprend le craquage en présence d'eau.
